(19) Europäisches Patentamt
European Patent Office
Office européen des brevets

(11) Veröffentlichungsnummer: **0 414 182 B1**

(12) **EUROPÄISCHE PATENTSCHRIFT**

(45) Veröffentlichungstag der Patentschrift: **13.07.94**

(51) Int. Cl.[5]: **G01N 33/18**

(21) Anmeldenummer: **90115907.9**

(22) Anmeldetag: **20.08.90**

# (54) Vorrichtung zur Bestimmung der biochemischen Sauerstoffverbrauchsrate und deren Verwendung.

(30) Priorität: **23.08.89 DE 8910097 U**

(43) Veröffentlichungstag der Anmeldung:
**27.02.91 Patentblatt 91/09**

(45) Bekanntmachung des Hinweises auf die Patenterteilung:
**13.07.94 Patentblatt 94/28**

(84) Benannte Vertragsstaaten:
**DE**

(56) Entgegenhaltungen:
**DE-A- 2 163 612**
**DE-A- 2 952 343**
**DE-A- 3 128 439**
**FR-A- 2 418 201**
**FR-A- 2 598 834**
**US-A- 3 810 738**

(73) Patentinhaber: **Forschungszentrum Jülich GmbH**
**Wilhelm-Johnen-Strasse**
**D-52425 Jülich(DE)**

(72) Erfinder: **Tappe, Wolfgang**
**Johannestrasse 32**
**D-5170 Jülich(DE)**
Erfinder: **Keusen, Heinrich, Dr.**
**Lindenstrasse 1658**
**D-2725 Hemslingen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung bezieht sich auf eine Vorrrichtung zur Bestimmung der biochemischen Sauerstoffverbrauchsrate mit einem Reaktionsgefäß mit Einrichtungen zur Einführung von Mikroorganismen sowie von Substratflüssigkeit, das über einen $CO_2$-Absorber mit einem elektrolvtischen Sauerstofferzeuger und einem Druckindikator verbunden ist und einem Registriergerät für die in der abgesperrten Strecke Reationsgefäß - $CO_2$-Absorber - Sauerstofferzeuger - Druckindikator erzeugten Sauerstoffmengen.

Die biochemische Sauerstoffverbrauchsrate eines Systems aus Mikroorganismen und Substrat, wie insbesondere Bakteriensuspension und Nährlösung gibt einen Anhalt für die Aktivität der Mikroorganismen einerseits und (bei bekannten Mikroorganismen bekannter Konzentration) auf den Gehalt an biologisch abbaubaren Inhaltstoffen. Sie dient insbesondere im Bereich der aeroben Abwasserreinigung zur Ermittlung der Belebstschlammaktivität sowie der Höhe der Belastung des zu reinigenden Abwassers.

So ist aus der DE 29 52 343 A1 ein Verfahren zur Bestimmung des Sauerstoffverbrauchs in Belebungsbecken bekannt, bei dem die "Sauerstoffzehrung" in einer bestimmten Menge Abwasser/Schlammgemisch mittels einer Sauerstoffelektrode gemessen wird.

In der DE 21 63 612 A1 wird ein Verfahren zur Überwachung des Gehalts an biologisch abbaubarer Substanz in einer Probenflüssigkeit, insb. zur BSB-Bestimmung von Abwasser, mittels einer Mikroorganismenkultur beschrieben, die kontinuierlich mit Probenflüssigkeit und Sauerstoff zur Aufrechterhaltung etwa konstanter Lebensbedingungen versorgt wird und in der die sich einstellende Sauerstoffkonzentration ermittelt wird.

Als deutsches Einheitsverfahren (DEV 1988 ) ist die Bestimmung der "Sauerstoffverbrauchsrate" als Parameter für die Aktivität von Belebtschlämmen bekannt. Bei diesem Verfahren wird ein synthetisches Abwasser zu einer Belebtschlammprobe hinzugegeben, die anschließend 30 Minuten belüftet wird. Danach wird in einem geschlossenen, blasenfrei gefüllten Gefäß die Abnahme der Konzentration des gelösten Sauerstoffes mit einer Sauerstoffsonde gemessen. Aus der Abnahmegeschwindigkeit wird die "Sauerstoffverbrauchsrate" berechnet.

Bei dieser Bestimmung steht nur der gelöste Sauerstoff zur Verfügung (der sich sehr schnell erschöpft), so daß nur eine recht globale Kurzzeit-Aktivtätsermittlung resultiert. Ferner erscheint die Festsetzung einer 30 minütigen Vorbelüftungszeit sehr willkürlich. Einflüsse wie Substratmangel oder Herabsetzung der Aktivität durch Hemmstoffe können mit diesem Verfahren nur ungenügend berücksichtigt werden.

Im Gegensatz zu diesem Verfahren bieten manometrische Verfahren nach Warburg, wie sie z. B. in modifizierter Form mit dem Sapromaten® der Fa. Voith durchgeführt werden (siehe z.B. FR 24 18 201 A1), die Möglichkeit, den biochemischen Sauerstoffverbrauch nicht nur über eine kurze Zeitspanne zu erfassen, sondern den Verlauf über einen längeren Zeitraum zu verfolgen. Dieses Gerät (Sapromat® ) wird hauptsächlich zur Messung des biochemischen Sauerstoffverbrauches nach 5 Tagen ($BSB_5$) eingesetzt.

Zu einer Meßeinheit des Sapromaten® gehören je ein Reaktionsgefäß, ein Sauerstofferzeuger und ein Druckindikator, die durch Kunststoffschläuche miteinander verbunden und in einem temperierten Wasserbad untergebracht sind. Dieses geschlossene Meßsystem ist unabhängig von Luftdruckschwankungen. Die zu untersuchende Probe wird im Reaktionsgefäß durch einen Magnetrührer umgewälzt, so daß die zum Abbau der Abwasserlaststoffe erforderliche Sauerstoffmenge aus dem darüberliegenden Gasraum in das Substrat eindringen kann. Durch den Gaswechsel ($O_2$-Eintrag, $CO_2$-Produktion mit anschließender $CO_2$-Absorption) entsteht ein Unterdruck, woraufhin der Druckindikator anspricht und über einen Schalterstärker die Sauerstofferzeugung bis zum Druckausgleich in Tätigkeit setzt. Der für die Sauerstofferzeugung aufgewandte Elektrolysestrom wird dabei in jedem Meßzeitpunkt als Maß für die von der Biomasse verbreuchte Sauerstoffmenge genommen.

Dieses für die Langzeituntersuchungen brauchbare Gerät ist jedoch für die kontinuierliche Bestimmung der biochemischen Sauerstoffverbrauchsrate über einen kurzen Zeitraum von z. B. 1 Stunde weniger geeignet:

Ziel der Erfindung ist daher ein diesbezüglich verbessertes Gerät.

Die erfindungsgemäße Vorrichtung der eingangs genannten Art ist zu diesem Zweck gekennzeichnet durch eine Sauerstoffmeßsonde im Reaktionsgefäß zur Ermittlung des Gelöstsauerstoffs in demselben und einen mit der Meßsonde verbundenen Meß- und Steuerungsrechner als Registriergerät, der über die zeitliche Gelöstsauerstoffänderung korrigierte Sauerstoffverbrauchsraten angibt.

Es wurde nämlich festgestellt, daß die mit dem Sapromaten® ermittelte Sauerstoffverbrauchsrate bei Kurzzeitmessungen und insbesondere hoher Aktivität der Mikroorganismen nicht dem wahren Wert entspricht, da der Gelöstsauerstoff im Reaktionsgefäß ein Defizit gegenüber dem Sättigungswert bei Druckausgleich aufweist. Aus diesem Grunde wird erfindungsgemäß neben dem elektroytisch ermittelten Wert der Sauerstoffverbrauchsrate der Gelöstsauerstoff in der Reaktionsflüssigkeit überwacht und seine zeitliche

Änderung als Korrekturglied bei der Feststellung des wahren Sauerstoffverbrauchswerts berücksichtigt.

Vorzugsweise wird zusätzlich für eine fortlaufende Gasumwälzung in dem geschlossenen Kreis vom Reaktionsgefäß über den $CO_2$-Absorber, die Druckmeßzelle und die Elektrolysezelle zum Reaktionsgefäß zurück gesorgt, wofür insbesondere eine Schlauchpumpe oder eine Membranpumpe vorgesehen wird. (In diesem Kreis sind vorzugsweise auch Einrichtungen für die Einschleusung von Substratlösung bzw. Abwasser oder Normnährlösung einbezogen.)

Durch diese Gasumwälzung wird das $O_2$-Konzeintrationsgefälle von der Elektrolysezelle zum Reaktionsgefäß abgebaut und das Sauerstoffdefizit im Reaktionsgefäß gemindert. Schließlich wird durch den Gaskreis auch die $CO_2$-Absorption aus der Gasphase durch kontinuierliches Hindurchleiten des Gasstroms durch eine $CO_2$-Absorberstrecke beschleunigt, so daß Meßfehler durch verzögerte $CO_2$-Absorption vermindert sind. Für die $CO_2$-Absorption eignet sich bekanntlich Natronkalk, insb. in einer Waschflasche, jedoch wären auch Carbonat-Puffersysteme (z.B. Carbonat/Bicarbonat-Mischungen)brauchbar.

Der Eintrag von Sauerstoff in die Flüssigkeit des Reaktionsgefäßes hängt schließlich von der Aktivität des Rührers in diesem Gefäß ab: vorzugsweise wird daher eine vom $O_2$-Defizit abhängende Steuerung der Rührgeschwindigkeit mittels des in der Apparatur vorhandenen Meß- und Steuerungsrechners vorgesehen.

Nachfolgend wird die Erfindung anhand eines Ausführungsbeispiels unter Bezugnahme auf die angefügten Zeichnungen näher erläutert.

Es zeigen schematisch:

| | |
|---|---|
| Figur 1 | Eine erfindungsgemäße Vorrichtung im Blockschaltbild; |
| Figur 2 | das Reaktionsgefäß der Vorrichtung von Figur 1 in vergrößerter Form; |
| Figur 3 | eine Labor-Ausführung der erfindungsgemäßen Vorrichtung und |
| Figur 4 u. 5 | BSR-Kurven, die mit einem solchen Gerät erhalten wurden. |

Gemäß Figur 1 ist bei der erfindungsgemäßen Vorrichtung analog zum Sapromaten® ein Reaktionsgefäß 1, ein Sauerstofferzeuger 2, eine Druckmeßzelle 3 und ein Meß-und Steuergerät 4 vorgesehen sowie eine in der Figur nicht gezeigte Thermostatisierung, z.B. mittels eines Wasserbades.

Das Reaktionsgefäß 1 ist gegenüber dem des Sapromaten® abgewandelt und umfaßt typischerweise: eine $O_2$-Meßsonde 5 zur fortlaufenden Überwachung des Gelöstsauerstoffs sowie Anschlüsse 6, 7 und 8 für den Zulauf von Mikroorganismensuspension (6), von Abwasser- bzw. Substratlösung (7) sowie den Gaszustrom (8) der Gasumwälzung mit Gasausgang 9. Über den Anschluß 10 werden Abwasser/Mikroorganismensuspension abgelassen. Ein weiterer Anschluß 11 in Verbindung mit einem Vorratsgefäß 12 für Normnährlösung mit Absperrventil 13 dient der weiter unten angegebenen Ermittlung der Anwesenheit von Hemmstoffen.

Typischerweise ist das Reaktionsgefäß zylindrisch und hat z. B. ein Volumen von 300 ml. Die $O_2$-Meßsonde ist über Flansche gasdicht eingebracht. Eine weitere Durchführung kann für eine pH-Meßsonde vorgesehen sein.

Die Befüllung und Entleerung erfolgt vorzugsweise zentralgesteuert über entsprechende Pumpen.

In Abwandlung des Sapromaten®, bei dem die $CO_2$-Absorption im oberen Bereich des Reaktionsgefäßes angeordnet ist, befindet sich ein $CO_2$-Absorber 14 in dem von der Gasförderpumpe 15 (Förderleistung z. B. 100 ml/min) angetriebenen Gaskreis für die Gasumwälzung. Figur 1 zeigt ferner Förderpumpen 16, 17, 18 (z. B. Schlauchpumpen mit einer Förderleistung von etwa 200 ml/min, computergeregelte Förderzeit bzw. -menge) für die Zufuhr von Mikroorganismen, Abwasser und Nährlösung sowie 19 für die Entnahme von Mikroorganismen/Abwasser aus dem Reaktionsgefäß 1. Mit 20 ist ein Abwasservorratsbehälter (von z. B. 70 ml) mit Absperrventil 20' (z. B. Magnetventil) bezeichnet. Der Behälter 20 ist in den Gaskreis einbezogen, so daß aus 20 druckfrei im geschlossenen System Abwasser (über 17) oder Normnährlösung (über 18 mit zugehörigem Vorratsgefäß 12') in das Reaktionsgefäß 1 abgefüllt werden kann. Alternativ zur Einschleusung von Normnährlösung über die Pumpe 18 kann auch, wie in Figur 2 angedeutet, ein unmittelbar angeschlossenes Vorratsgefäß 12 für Normnährlösung vorgesehen sein, das dann zur Entlüftung in den Gaskreis integriert wird.

21 ist ein computergesteuertes Druckausgleichsventil und 22, 22' ein vom Rechner gesteuerter Magnetrührer für das Gefäß 1 (mit konstanter, einstellbarer Drehzahl von z. B. 150, 200, 250 Upm oder elektronischer Drehzahlregelung abhängig vom Sauerstoffdefizit). Der zentrale Meß- und Steuerungsrechner 23 (z.B. ein Hybrid-Recorder der Fa.Chessell) dient neben der Ventil- und Pumpensteuerung der Meßwertverarbeitung:

Er empfängt dafür die von der $O_2$-Meßsonde mit Meßverstärker und Signalausgang ($T_{90}$ < 30 sec.; $T_{90}$ = Zeitspanne bis zum Erreichen von 90 % des Endwertes bei einer raschen Änderung der $O_2$-Konzentration) herkommenden Signale für die Gelöstsauerstoffkonzentration sowie die vom Meß- und Steuergerät der Elektrolysezelle abgegebenen BSV-Werte (jeweils in mg/l) und berechnet danach die biochemische Sauerstoffverbrauchsrate (BSR) nach der Formel:

$$BSR = \frac{BSV(t_{x1}) - BSV(t_o)}{t_{x1} - t_o} - \frac{O_2(t_{x2}) - O_2(t_o)}{t_{x2} - t_o}$$

Für die Differenzierung der BSV-Werte wird die Zeitspanne dt durch aufeinanderfolgende Einschaltzeitpunkte (oder Ausschaltzeitpunkte) bestimmt. Zur Differenzierung der $O_2$-Werte ist in der Regel $\Delta t$ (BSV) > $\Delta t$ ($O_2$) > 0,1 min zu wählen. Der Meß- und Steuerrechner zeichnet dann die BSR-Kurve aus.

Bei der vorstehend beschriebenen Vorrichtung erfolgt die Messung und Regelung in folgender Weise:
Zu Beginn der Messung wird der Vorratsbehälter 20 mit Abwasser und/oder Nährlösung und das Reaktionsgefäß 1 mit der Mikroorganismensuspension befüllt. Während des Befüllungsvorganges ist das Druckausgleichsventil 21 geöffnet und das Absperrventil des Vorratsbehälters 20' geschlossen. Die Flüssigkeitsvolumina sind je nach den Anforderungen frei wählbar, lediglich die Summe beider Volumina muß bekannt sein. Sie geht in die Umrechnung des volumenbezogenen, elektrolytisch produzierten Sauerstoffs (BSV) ein. Die Meß- und Steuereinheit 4 berechnet den BSV auf der Grundlage von 250 ml Probenvolumen. (Bei entsprechender Programmierung von 23 kann 4 entfallen.)Weicht das Probenvolumen Vx von dem Sollvolumen Vo ab, sind die Meßwerte mit dem Faktor Vx/Vo zu multiplizieren.

Nach dem Befüllungsvorgang wird das Druckausgleichsventil 21 geschlossen. In einer "Angleichphase" wird die Probe zwecks Temperaturangleichs und Einstellung des $CO_2/O_2$-Gleichgewichtes für eine Zeitspanne von mindestens 5 Minuten gerührt. Die Rührgeschwindigkeit wird auf einen empirisch festgelegten Wert von z. B. 150 UpM eingestellt. Die Steuer- und Recheineinheit 23 zeichnet kontinuierlich die $O_2$ - und BSV-Werte auf und berechnet die BSR-Werte. Während der Angleichphase wird die Rührgeschwindigkeit so eingestellt, daß die $O_2$-Konzentration mit einer Rate von z. B. 1 mg $O_2$/(l x min) ansteigt. Sobald ein Schwellenwert von z. B. 6 mg/l erreicht ist, wird die Rührgeschwindigkeit auf den aktuellen Wert festgesetzt.

Die Angleichphase wird nach Ablauf der festgeschriebenen Zeitspanne (z. B. 5 min) oder dann beendet, wenn über einen Zeitraum von z. B. einer Minute die $O_2$- und BSR-Werte konstante Werte (+/- 10 % vom Mittelwert) anzeigen. Sind beide Bedingungen erfüllt, öffnet sich das Absperrventil 20' und entläßt die Flüssigkeit in das Reaktionsgefäß 1. Typischerweise sinkt die $O_2$-Konzentration nach der Flüssigkeitszugabe auf Werte < 5 mg $O_2$/l. Sollte der Wert unter 1 mg/l sinken, ist die Rührgeschwindigkeit zu erhöhen (z. B. stufenweise um jeweils 20 %), um eine Sauerstoffunterversorgung zu vermeiden. Werden $O_2$-Minima von > 5 mg/l gemessen, kann ggf. die Rührgeschwindigkeit verringert werden (z. B. 20 %). Dies ist insbesondere dann notwendig, wenn die Elektrolysezelle 2 über einen längeren Zeitraum (> 2 min) permanent $O_2$ produziert. Das ist zu vermeiden, da es zu einem Minderbefund führt.

Werden trotz permanenter $O_2$-Nachlieferung $O_2$-Konzentrationen < 1 mg/l gemessen, ist die Meßkapazität des Systems überschritten. Die Messung wird dann beendet und ggf. wiederholt, wobei die Abwassermenge verringert und/oder die Mikroorganismenkonzentration verdünnt und/oder die Stromstärke der Elektrolysezelle erhöht wird.

Im Normalfall wird die Messung nach einem definierten Zeitraum (z. B. 1 h) oder nach Absinken der Aktivität unter einen Sollwert abgeschlossen Als Sollwert kann beispielsweise eine 10 minütige Zeitspanne definiert werden, in der BSR-Werte gemessen werden, die höchstens 10 % über den vor Substratzugabe gemessenen BSR-Werten liegen.

Nach Beendigung der Messung wird das Druckausgleichsventil geöffnet. Je nach Anforderungen wird ein neuer Abwasserschub in den Vorratsbehälter (Absperrventil geschlossen) gefördert und die Messung mit der gleichen Mikroorganismensuspension wiederholt. Andernfalls wird das Abwasser/Mikroorganismengemisch insgesamt aus dem Probengefäß gefördert.

Eine einfache Laborvariante der oben beschriebenen Vorrichtung wird in Fig. 3 gezeigt. Entgegen der in Fig. 1 beschriebenen automatischen Befüllung und Entleerung des Reaktionsgefäßes mittels ansteuerbarer Absperrventile und Pumpen erfolgen diese Vorgänge hierbei manuell. Meßprinzip, Reaktionsgefäß und $CO_2$-Absorption entsprechen Fig. 1.

Das Reaktionsgefäß 1 wird mittels Magnetrührer 22 durchmischt. Der Druckindikator 3 löst bei Druckabfall die Sauerstoffproduktion durch den Sauerstofferzeuger 2 aus. Die produzierte Sauerstoffmenge wird durch das Registriergerät 24 aufgezeichnet und kontinuierlich dem Rechner 25 zugeführt. Die im Reaktionsgefäß 1 befindliche Sauerstoffelektrode 5 leitet dem Rechner 25 über den Meßverstärker 26 die aktuelle Sauerstoffkonzentration in der Probenlösung zu, deren zeitliche Änderung das Korrekturglied bei der Feststellung des wahren Sauerstoffverbrauchswertes darstellt.

Mittels der durch die Spannungsquelle 27 gespeisten Vakuumpumpe 28 wird eine fortlaufende Gasumwälzung in dem geschlossenen Kreis vom Reaktionsgefäß über den $CO_2$-Absorber 14, die Druckmeßzelle 3 und den Sauerstofferzeuger (Elektrolysezelle) 2 zum Reaktionsgefäß 1 zurück gesorgt. Die Einschleusung von Substratlösung bzw. Normnährlösung erfolgt über das Septum 29.

Die beschriebene Vorrichtung eignet sich insbesondere für die Steuerung der Substratzufuhr bei biotechnologischen Prozessen in Abhängigkeit von der Sauerstoffzehrung.

Für Untersuchungen von Hemmstoffen ist die serielle Messung der Aktivität der Testorganismen nach Zugabe einer Nährlösung und die anschließende Untersuchung einer Mischung aus Nährlösung und hemmstoffenthaltendem Abwasser vorgesehen. Zu diesem Zweck wird in der ersten Meßphase die Nährlösung in dein Vorratsbehälter gefördert und die Aktivität der Mikroorganismen nach Zugabe der Nährlösung untersucht ("Normalaktivität"). Nach Abschluß der ersten Meßphase wird außer der Nährlösung auch das hemmstoffhaltige Abwasser in den Vorratsbehälter gefördert und die Messung wiederholt. Die Meßwerte der zweiten Meßphase werden mit den Werten der ersten Phase verglichen. Bei Anwesenheit von Hemmstoffen im Abwasser wird der Erwartungswert nach Zugabe von Abwasser nicht ereicht, woraus auf die Hemmwirkung geschlossen werden kann. Besonders empfindlich reagieren bei der Hemmstoffprüfung Nitrifikanten.

Ein typischer Verlauf solcher BSR-Kurven ist in Figur 4 und Figur 5 wiedergegeben, die anhand von mit Modellabwasser gefütterten Belebtschlammproben erhalten wurden:
Die vor der Substratzugabe (S) gemessenen Werte entsprechen dem Grundsauerstoffverbrauch des "ausgehungerten" Belebtschlammes. Nach Substratzugabe steigt die Sauerstoffverbrauchsrate an und erreicht nach einer Dauer von ca. 30 Minuten (Zehrungsphase) wieder die Grundrate. Während der anschließenden "Hungerphase" änderten sich diese Werte nicht. Die Fläche unterhalb der Kurve während der Zehrungsphase ist charakteristisch für die Abwasserbelastung und die aktuelle Aktivität der Mikroorganismen.

Es können dabei Phasen verschieden hoher Aktivitäten unterschieden werden. Insbesondere ist die Nitrifikationsphase anhand dieser Meßkurve deutlich erkennbar ("Nitrifikationsplateau"). In Figur 5 wird exemplarisch der Einfluß eines Hemmstoffes (Dimethyldisulfid) auf die Atmungsaktivität dargestellt. Insbesondere die Nitrifikanten reagieren sensitiv auf diesen Hemmstoff. Das im Modellabwasser enthaltene Ammonium wird infolge gehemmter Nitrifikation nicht mehr in gleicher Weise wie in dem in Figur 4 dargestellten Versuch oxidiert (kein "Nitrifikationsplateau").

**Patentansprüche**

**1.** Vorrichtung zur Bestimmung der biochemischen Sauerstoffverbrauchsrate mit einem Reaktionsgefäß (1) mit Einrichtungen zur Einführung von Mikroorganismen sowie von Substratflüssigkeit, das über einen $CO_2$-Absorber (14) mit einem elektrolytischen Sauerstofferzeuger (2) und einem Druckindikator (3) verbunden ist und einem Registriergerät für die in der abgesperrten Strecke Reaktionsgefäß - $CO_2$-Absorber - Sauerstofferzeuger - Druckindikator erzeugten Sauerstoffmengen,
**gekennzeichnet durch**
eine Sauerstoffmeßsonde (5) im Reaktionsgefäß (1) zur Ermittlung des Gelöstsauerstoffs in demselben und einen mit der Meßsonde verbundenen Meß- und Steuerungsrechner (23) als Registriergerät, der über die zeitliche Gelöstsauerstoffänderung korrigierte Sauerstoffverbrauchsraten angibt.

**2.** Vorrichtung nach Anspruch 1,
**gekennzeichnet durch**
einen Meß- und Steuerungsrechner (23), der fortlaufend die biochemische Sauerstoffverbrauchsrate (BSR) nach der Formel

$$BSR = \frac{BSV(t_{x1})-BSV(t_o)}{tx1-t_o} - \frac{O_2(t_{x2})-O_2(t_o)}{t_{x2}-t_o}$$

ermittelt und registriert, in der BSV ($t_{x1}$) und BSV($t_o$) der zu den Zeiten $t_{x1}$ bzw. $t_o$ gemessene biochemischen Sauerstoffverbauch und $O_2$ ($t_{x2}$) bzw. $O_2$ ($t_o$) die gemessenen Gelöstsauerstoffwerte zu den Zeiten $t_{x2}$ bzw. $t_o$ sind, insbesondere mit einem Meßwertnahmeprogramm zu den Zeitpunkten $t_{x2}$, $t_{x1}$ und $t_o$ derart, daß $(t_{x1} - t_o) > (t_{x2} - t_o)$.

3. Vorrichtung nach Anspruch 1 oder 2,
**gekennzeichnet durch**
Einrichtungen zur Gasumwälzung im Leitungskreis Reaktionsgefäß (1) - $CO_2$-Absorber (14) - Druckmeßzelle (3) - Elektrolysezelle (2) - Reaktionsgefäß (1).

4. Vorrichtung nach Anspruch 3,
**gekennzeichnet durch**
eine Schlauchpumpe (15) im Leitungskreis zur Gasumwälzung.

5. Vorrichtung nach einem der vorangehenden Ansprüche,
**gekennzeichnet durch**
eine Natronkalk enthaltende Leitungsstrecke, insbesondere in Form einer Waschflasche als $CO_2$-Absorber (14).

6. Vorrichtung nach einem der vorangehenden Ansprüche,
**gekennzeichnet durch**
Einrichtungen (18, 20 bzw. 11 - 13) für die Einschleusung von Normnährlösung in das Reaktionsgefäß (1) für die Durchführung von Hemmstofftests.

7. Vorrichtung nach einem der vorangehenden Ansprüche,
**gekennzeichnet durch**
eine vom Sauerstoffdefizit abhängige Drehzahlsteuerung für den im Reaktionsgefäß (1) vorgesehenen Rührer (22, 22').

8. Verwendung der Vorrichtung nach einem der vorangehenden Ansprüche,
für die Steuerung der Substratzufuhr bei biotechnologischen Prozessen in Abhängigkeit von der Sauerstoffzehrung.

9. Verwendung der Vorrichtung nach einem der vorangehenden Ansprüche,
zur Ermittlung von Hemmstoffen in Abwasserreinigungsanlagen einer Normabwassermischtechnik.

**Claims**

1. An apparatus for determining the biochemical oxygen demand rate, with a reaction vessel (1) with devices for introducing microorganisms and substrate liquid, which reaction vessel is connected via a $CO_2$ absorber (14) to an electrolytic oxygen generator (2) and a pressure indicator (3), and with a recording unit for the amounts of oxygen produced in the closed section comprising reaction vessel - $CO_2$ absorber - oxygen generator - pressure indicator, characterised by an oxygen measuring head (5) in the reaction vessel (1) for determining the dissolved oxygen in the same and by a measurement and control computer (23) connected as the recording unit to the measuring head, which computer indicates corrected oxygen demand rates via the temporal variation of the dissolved oxygen.

2. An apparatus according to claim 1, characterised by a measurement and control computer (23) which continuously determines and records the biochemical oxygen demand rate (BSR) according to the formula

$$BSR = \frac{BSV(t_{x1}) - BSV(t_0)}{t_{x1} - t_0} - \frac{O_2(t_{x2}) - O_2(t_0)}{t_{x2} - t_0}$$

where BSV ($t_{x1}$) and BSV ($t_0$) are the biochemical oxygen demands measured at times $t_{x1}$ and $t_0$ respectively, and $O_2$ ($t_{x2}$) and $O_2$ ($t_0$) are the measured dissolved oxygen values at times $t_{x2}$ and $t_0$ respectively, particularly with a program for taking readings at times $t_{x2}$, $t_{x1}$ and $t_0$ such that $(t_{x1} - t_0) > (t_{x2} - t_0)$.

3. An apparatus according to claim 1 or 2, characterised by devices for the circulation of gas in the line circuit comprising reaction vessel (1) - $CO_2$ absorber (14) - pressure measuring cell (3) -electrolysis cell (2) - reaction vessel (1).

4. An apparatus according to claim 3, characterised by a peristaltic pump (15) in the line circuit for gas circulation.

5. An apparatus according to any one of the preceding claims, characterised by a line section containing soda lime, particularly in the form of a wash-bottle, as a $CO_2$ absorber (14).

6. An apparatus according to any one of the preceding claims, characterised by devices (18, 20; 11 - 13) for transferring standard nutrient solution into the reaction vessel (1) for performing inhibitor tests.

7. An apparatus according to any one of the preceding claims, characterised by an oxygen deficit-dependent speed control system for the stirrers (22, 22') provided in the reaction vessel (1).

8. A use of the apparatus according to any one of the preceding claims for controlling the supply of substrate in biotechnological processes in accordance with the provision of oxygen.

9. A use of the apparatus according to any one of the preceding claims for determining inhibitors in sewage purification installations of a standard sewage mixing technique.

**Revendications**

1. Dispositif pour la détermination du taux de consommation d'oxygène biochimique, comportant un réacteur (1) pourvu d'installations pour l'amenée de micro-organismes ainsi que d'un substrat liquide, qui est relié à un générateur électrolytique d'oxygène (2) et à un indicateur de pression (3) par l'intermédiaire d'un absorbeur de $CO_2$ (14), ainsi qu'un appareil d'enregistrement pour les quantités produites d'oxygène dans le circuit fermé cuve de réaction - absorbeur de $CO_2$ - générateur d'oxygène - indicateur de pression, caractérisé par une sonde de mesure d'oxygène (5) située dans le réacteur pour déterminer la quantité d'oxygène dissous dans celui-ci, et un appareil de mesure et de commande (23), comme appareil d'enregistrement, qui est relié à la sonde de mesure et indique les taux de consommation d'oxygène en fonction de la modification dans le temps de la quantité d'oxygène dissous.

2. Dispositif selon la revendication 1, caractérisé par un appareil de mesure et de commande (23) qui détermine et enregistre en continu le taux de consommation d'oxygène biochimique (BSR) selon la formule :

$$BSR = \frac{BSV(t_{x1})-BSV(t_0)}{t_{x1}-t_0} - \frac{O_2(t_{x2})-O_2(t_0)}{t_{x2}-t_0}$$

dans laquelle $BSV(t_{x1})$ et $BSV(t_0)$ représentent la consommation d'oxygène biochimique mesurée respectivement aux moments $t_{x1}$ et $t_0$, et $O_2(t_{x2})$ et $O_2(t_0)$ représentent les valeurs mesurées d'oxygène dissous respectivement aux moments $t_{x2}$ et $t_0$, en particulier avec un programme d'enregistrement des valeurs mesurées aux moments $t_{x2}$, $t_{x1}$ et $t_0$ d'une manière telle que $(t_{x1}-t_0) > (t_{x2}-t_0)$.

3. Dispositif selon la revendication 1 ou 2, caractérisé par des installations de circulation de gaz dans le circuit fermé réacteur (1) - absorbeur de $CO_2$ (14) - capteur de pression (3) - cellule électrolytique (2) - réacteur (1).

4. Dispositif selon la revendication 3, caractérisé par une pompe tubulaire (15) dans le circuit fermé de circulation de gaz.

5. Dispositif selon une des revendications précédentes, caractérisé par une tuyauterie contenant de la chaux sodée, en particulier sous la forme d'un barboteur à gaz, comme absorbeur de $CO_2$ (14).

6. Dispositif selon une des revendications précédentes, caractérisé par des installations (18, 20 ou 11-13) pour l'injection d'un bouillon de culture standard dans le réacteur (1) pour la mise en oeuvre d'essais d'inhibiteurs.

7. Dispositif selon une des revendications précédentes, caractérisé par une commande de vitesse, dépendante du déficit en oxygène, pour l'agitateur (22, 22') prévu dans le réacteur (1).

8. Utilisation du dispositif selon une des revendications précédentes pour le réglage de l'amenée de substrat dans des opérations biotechnologiques en fonction de la consommation d'oxygène.

9. Utilisation du dispositif selon une des revendications précédentes pour la détermination d'inhibiteurs dans des installations de purification des eaux usées avec une technique standard de mélange des eaux usées.

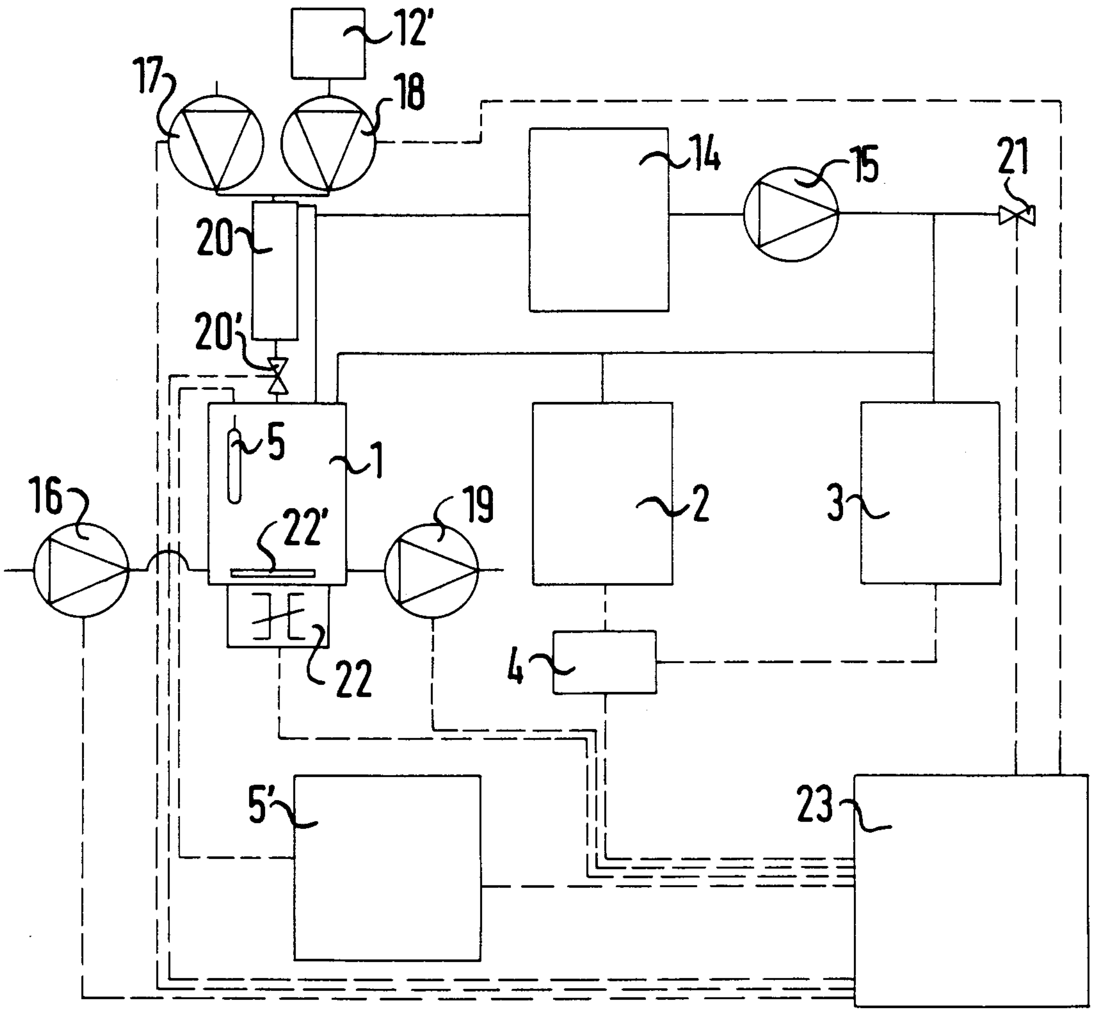


FIG. 1

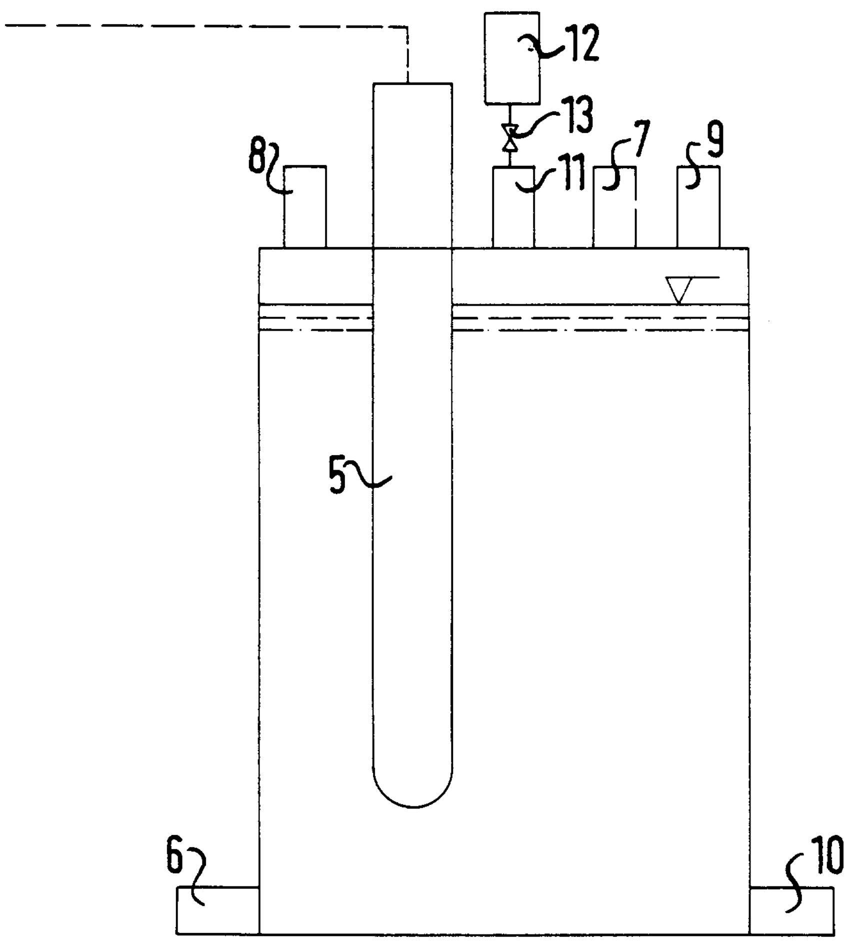


FIG. 2

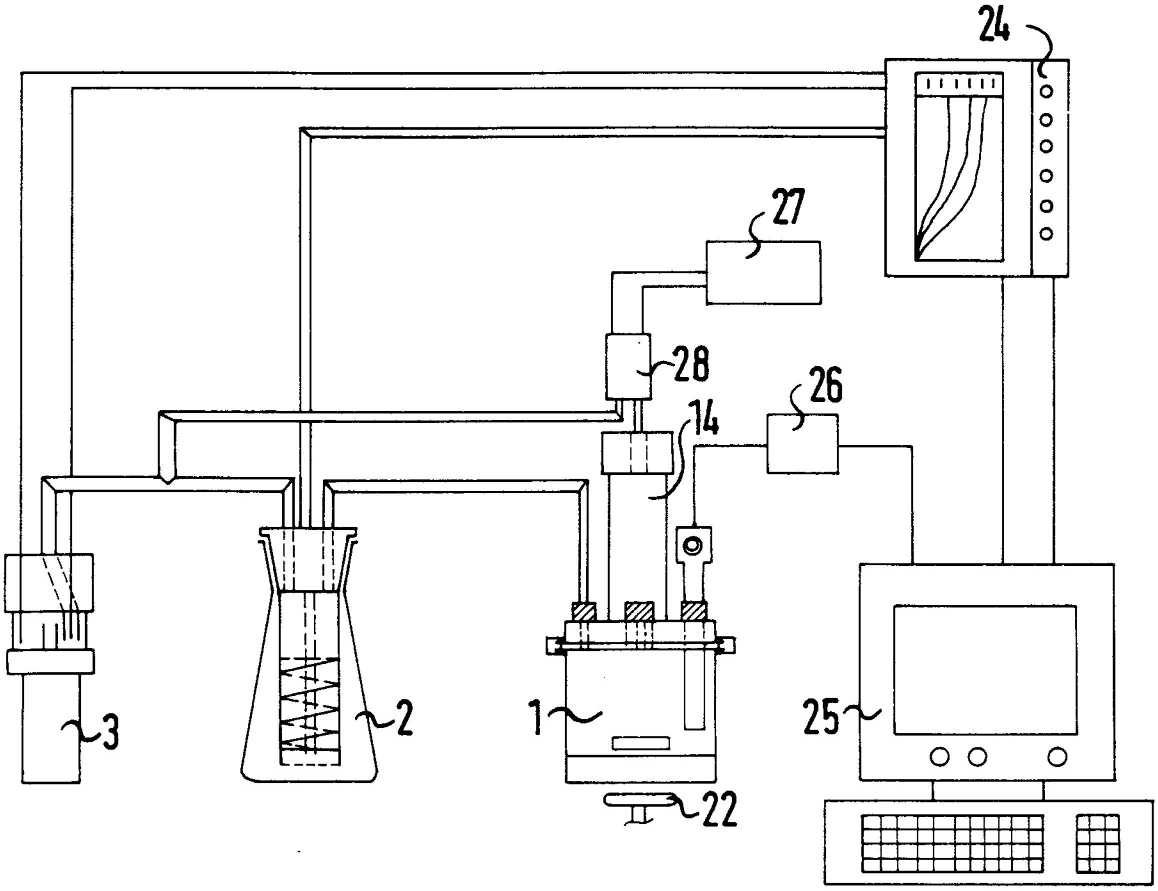


FIG. 3

FIG. 4

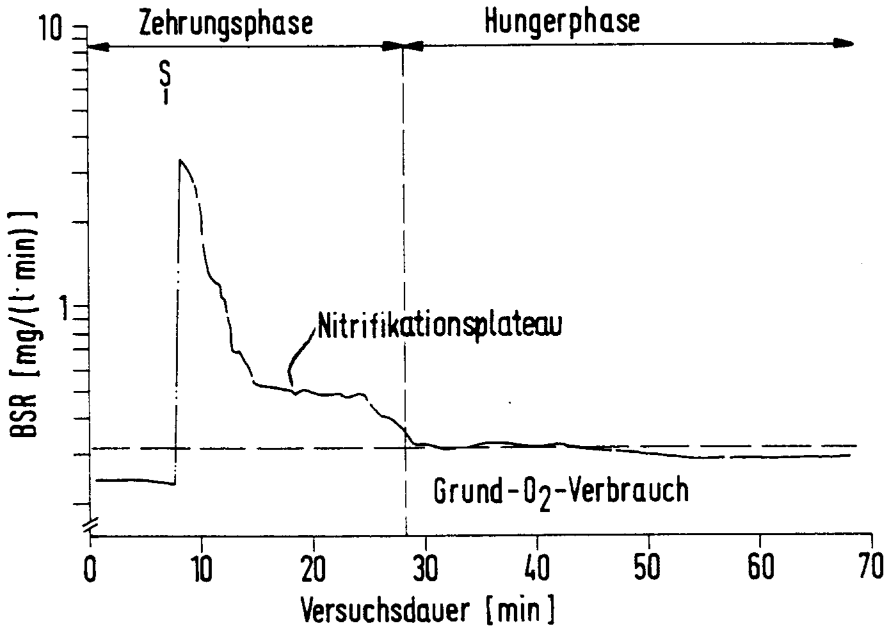
10
1
Zehrungsphase
Hungerphase
S
Nitrifikationsplateau
Grund-$O_2$-Verbrauch
BSR [mg/(l·min)]
0
10
20
30
40
50
60
70
Versuchsdauer [min]

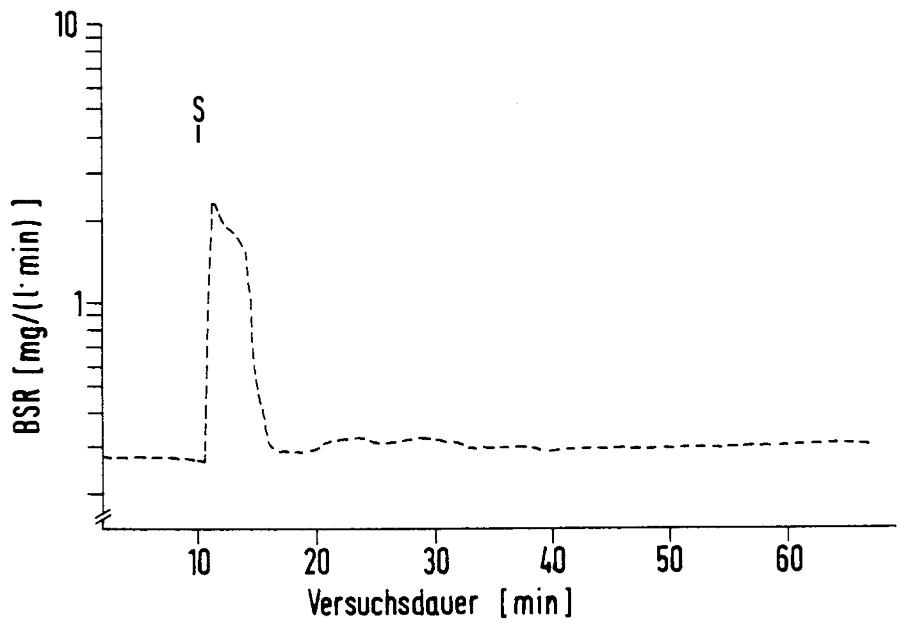
10
1
S
BSR [mg/(l·min)]
10
20
30
40
50
60
Versuchsdauer [min]

FIG. 5